# EUROPÄISCHE PATENTSCHRIFT

(11) **EP 2 846 852 B1**
(45) Veröffentlichungstag und Bekanntmachung des Hinweises auf die Patenterteilung: **19.04.2017**
(21) Anmeldenummer: 13726105.3
(22) Anmeldetag: 07.05.2013
(51) Int. Cl.: A61M 1/14, A61M 1/36

(54) **VORRICHTUNG ZUR EXTRAKORPORALEN BLUTBEHANDLUNG ZUM ERMITTELN EINER BLUTFLUSSRATE FÜR EINE EXTRAKORPORALE BLUTBEHANDLUNGSVORRICHTUNG**
EXTRACORPOREAL BLOOD TREATMENT DEVICE FOR ASCERTAINING A BLOOD FLOW RATE FOR AN EXTRACORPOREAL BLOOD TREATMENT DEVICE
DISPOSITIF DE TRAITEMENT EXTRACORPOREL DU SANG DE DÉTERMINATION D'UN DÉBIT SANGUIN DANS UN DISPOSITIF DE TRAITEMENT EXTRACORPOREL DU SANG

(30) Priorität: 10.05.2012 DE 102012009192; 10.05.2012 US 201261645103 P
(43) Veröffentlichungstag der Anmeldung: 18.03.2015
(73) Patentinhaber: Fresenius Medical Care Deutschland GmbH, 61352 Bad Homburg v.d.H. (DE)
(72) Erfinder: KOPPERSCHMIDT, Pascal, 97456 Dittelbrunn (DE); WEHMEYER, Wolfgang, 72076 Tübingen (DE)
(74) Vertreter: Oppermann, Frank
(86) Internationale Anmeldenummer: PCT/EP2013/001355
(87) Internationale Veröffentlichungsnummer: WO 2013/167264

(56) Entgegenhaltungen:
- EP-A1- 0 834 329
- EP-A2- 0 240 101
- US-A- 5 507 723
- US-A1- 2010 168 641
- US-B1- 6 200 485

## Beschreibung

Die Erfindung betrifft eine Vorrichtung zur extrakorporalen Blutbehandlung (siehe z.B. US20100168641), bei der Blut in einem extrakorporalen Blutkreislauf durch eine arterielle Blutleitung in die Blutkammer eines durch eine semipermeable Membran in die Blutkammer und eine Dialysierflüssigkeitskammer getrennten Dialysators strömt und durch eine venöse Blutleitung aus der Blutkammer strömt.

Es sind verschiedene Verfahren zur extrakorporalen Blutbehandlung bekannt, beispielsweise die Hämodialyse, die Hämofiltration sowie die Kombination aus beiden Verfahren, die als Hämodiafiltration bezeichnet wird. Während der Hämodialysebehandlung strömen das Blut des Patienten und eine Dialysierflüssigkeit vorzugsweise im Gegenstrom jeweils mit einer vorgegebenen Flussrate entlang der semipermeablen Membran, die den Dialysator in die Blut- und Dialysierflüssigkeitskammer trennt.

Zur Optimierung der Blutbehandlungsverfahren ist es bekannt, für die Blutbehandlung charakteristische Kenngrößen zu bestimmen. Zu diesen charakteristischen Kenngrößen gehören insbesondere die Parameter, die die Austauschleistung des Dialysators beschreiben. Die Austauschleistung des Dialysators kann durch die Clearance beschrieben werden, die für einen bestimmten Stoff dasjenige virtuelle Blutvolumen bezeichnet, das pro Minute durch den Dialysator vollkommen von diesem Stoff befreit wird. Die Dialysance ist ein weiterer Begriff zur Bestimmung der Leistungsfähigkeit des Dialysators, bei dem auch die Konzentration des am Stoffaustausch im Dialysator beteiligten Stoffes in der Dialysierflüssigkeit berücksichtig wird.

Die Clearance bzw. Dialysance hängen wiederum entscheidend von der vorgegebenen Blutflussrate ab, mit der das Blut des Patienten durch die Blutkammer des Dialysators strömt. Die Clearance nimmt dabei mit steigender Blutflussrate zu. Daher sollte die Blutbehandlung mit einer möglichst hohen Blutflussrate erfolgen. In der Praxis zeigen sich aber Grenzen für die Erhöhung des Blutflusses.

Eine obere Grenze für den Blutfluss stellt der Gefäßzugang dar, der den arteriellen Zulauf- und venösen Rücklauf druck in den Blutleitungen begrenzt. Eine Erhöhung der Blutflussrate, mit der die Blutpumpe das Blut durch die Blutleitungen fördert, führt zu einer Erhöhung des Saugdrucks der Blutpumpe, wodurch das Gefäß kollabieren oder die Punktionsnadel sich an der Gefäßwand ansaugen kann. In diesem Fall wird ein arterieller Druckalarm ausgelöst. In Abhängigkeit von der Beschaffenheit des Gefäßzugangs sind die Grenzwerte für den Blutfluss von Patient zu Patient verschieden. Auch bei dem gleichen Patienten ergeben sich unterschiedliche Grenzwerte für den Blutfluss, da beispielsweise neu angelegte Shunts (arterio-venöse Fistel) in der Regel noch relativ instabil sind und keine zu hohen Drücke vertragen. Gängige Maximalwerte für die Zulaufdrücke liegen im Bereich von -200 mmHg bis -150 mmHG, für die Rücklaufdrücke werden bis zu +150 mmHg bis +200 mmHg toleriert. Die Höhe des Blutflusses ist auch bei den Punktionskanülen von entscheidender Bedeutung, da Kanülen mit einem kleinen Durchmesser einen höheren Strömungswiderstand als solche mit großem Durchmesser haben.

Bislang erfolgt die Einstellung des Blutflusses manuell unter Berücksichtigung der Vorgaben. Oftmals wird gewohnheitsbedingt ein Wert eingestellt, der sich in der Praxis bewährt hat, beispielsweise 300 ml/min, 350 ml/min oder 400 ml/min, obwohl das Blut und die Physiologie des Patienten grundsätzlich die Einstellung abweichender Werte erlaubt.

Die US 2010/0168641 A1 beschreibt eine Blutbehandlungsvorrichtung, die über eine Steuereinrichtung zur Einstellung der Flussrate der Blutpumpe verfügt. Die Steuereinrichtung überwacht den Druck in der Vene und stellt für die Flussrate der Blutpumpe in Abhängigkeit von dem gemessenen Druck einen Wert ein, bei dem ein Kollabieren der Vene nicht auftreten kann. Die Abhängigkeit der Blutflussrate von dem Druck in der Vene wird für die jeweilige Blutbehandlungsvorrichtung durch einen Algorithmus beschrieben, der in einem Speicher abgelegt wird.

Der Erfindung liegt die Aufgabe zu Grunde, eine Vorrichtung zur extrakorporalen Blutbehandlung zu schaffen, die eine Optimierung der vorzugebenden Blutflussrate im Sinne einer Maximierung der Austauschleistung des Dialysators vorsieht.

Die Lösung dieser Aufgaben erfolgt erfindungsgemäß mit den Merkmalen des Patentanspruchs 1. Die Gegenstände der Unteransprüche betreffen vorteilhafte Ausführungsformen der Erfindung.

Die erfindungsgemäße Vorrichtung sieht die Ermittlung einer Mehrzahl von für die extrakorporale Blutbehandlung, insbesondere den extrakorporalen Blutkreislauf charakteristischer Kenngrößen (Parameter) vor, wobei jeweils in Abhängigkeit von einer der charakteristischen Kenngrößen eine bestimmte Blutflussrate ermittelt wird. Das Grundprinzip der Erfindung liegt darin, aus der Mehrzahl der auf der Grundlage der charakteristischen Kenngrößen ermittelten Blutflussraten eine Blutflussrate zu wählen, die für die Blutbehandlung vorgegeben wird. Die vorzugebende Blutflussrate wird dabei auf der Grundlage eines vorgegebenen Algorithmus ausgewählt, der in einer auf einer Datenverarbeitungseinheit installierten Software oder einer Hardware implementiert werden kann. Dabei wird unter einem Algorithmus jede eindeutige Vorschrift verstanden, die eine automatische Auswahl der Blutflussrate erlaubt.

Bei der erfindungsgemäßen Vorrichtung zur extrakorporalen Blutbehandlung ist die Einrichtung zum Vorgeben der Blutflussrate derart ausgebildet, dass auf der Grundlage des vorgegebenen Auswahlkriteriums eine automatische Auswahl der jeweiligen Blutflussrate, erfolgt. Die Einrichtung zum Vorgeben der Blutflussrate weist eine separate Datenverarbeitungseinheit aus, die derart programmiert ist, dass auf der Grundlage des vorgegebenen Algorithmus die Blutflussrate ausgewählt wird, mit der die Mittel zum Fördern von Blut im extrakorporalen Blutkreislauf, beispielsweise die in der arteriellen Blutleitung angeordnete Blutpumpe, betrieben werden.

Die Einrichtungen zum Ermitteln der Blutflussraten in Abhängigkeit von den charakteristischen Kenngrößen können selbst Regeleinrichtungen sein, die den Blutfluss in Abhängigkeit von den betreffenden Parametern regeln. Dabei geben die einzelnen Regeleinrichtungen der Einrichtung zum Vorgeben der Blutflussrate Blutflussempfehlungen auf der Grundlage der unabhängig voneinander überwachten charakteristischen Kenngrößen. Auch die Einrichtung zum Vorgeben der Blutflussrate kann selbst wieder eine Regeleinrichtung sein, die den Blutfluss dann in Abhängigkeit von der betreffenden charakteristischen Kenngröße regelt, auf deren Grundlage die Einrichtung die Auswahl getroffen hat.

Der Algorithmus zur Auswahl der für die Blutbehandlung vorzugebenden Blutflussrate kann unterschiedliche Auswahlkriterien vorsehen. Vorzugsweise wird unter den empfohlenen Blutflussraten die Blutflussrate ausgewählt, bei der eine Blutbehandlung mit hoher Sicherheit durchgeführt werden kann. In der Praxis wird daher das Auswahlkriterium darin liegen, den niedrigsten Wert der empfohlenen Blutflussraten auszuwählen.

Bei einer bevorzugten Ausführungsform der Erfindung fließen in die Auswahl der optimalen Blutflussrate nicht nur die dynamisch in Abhängigkeit von den charakteristischen Größen ermittelten Blutflussraten, sondern auch vorgegebene statische Blutflussraten ein, die einen Maximalwert und/oder einen Minimalwert für den Blutfluss vorgeben. Dadurch ist es möglich, bei der Auswahl der optimalen Blutflussrate Minimal- bzw. Maximalwerte für den Blutfluss zu berücksichtigen. Dadurch wird die Sicherheit der Blutbehandlung weiterhin erhöht.

Für die Erfindung ist grundsätzlich von Bedeutung, welche charakteristischen Kenngrößen für die Ermittlung der einzelnen Blutflussraten herangezogen werden. Dies sind insbesondere die Parameter, die mit der Veränderung der Blutflussrate in einem engen Zusammenhang stehen. Insbesondere ist die Überwachung der die Stoffaustauschleistung des Dialysators beschreibenden Clearance und/oder die Überwachung des arteriellen Drucks in der arteriellen Blutleitung und/oder die Überwachung des venösen Drucks in der venösen Blutleitung vorgesehen. Die Ermittlung dieser für die Blutbehandlung charakteristischen Kenngrößen ist dem Fachmann bekannt. Einrichtungen zur Ermittlung dieser Parameter gehören zum Stand der Technik.

Eine weitere besonders bevorzugte Ausfühlarigsform sieht auch die Bestimmung einer für die Anzahl der im Blut befindlichen Mikroblasen charakteristischen Kenngröße vor, da ein bestimmtes Volumen der im Blut gelösten Mikroblasen nicht überschritten werden darf. Eine Einrichtung zur Ermittlung einer derartigen Kenngröße gehört ebenfalls zum Stand der Technik. Die Bildung von Mikroblasen (Kavitation) kann aufgrund des negativen arteriellen Drucks erfolgen. Weiterhin können Undichtigkeiten im Blutschlauchsystem in Verbindung mit einem gegenüber dem Außendruck negativen Blutdruck im Blutschlauch zur Bildung von Mikroblasen führen.

Bei einer weiteren besonders bevorzugten Ausführungsform gibt die Einrichtung zum Vorgeben der Blutflussrate als Startwert für die Blutbehandlung eine Blutflussrate vor, mit der eine vorausgehende Blutbehandlung des gleichen Patienten beendet worden ist. Grundsätzlich ist es aber auch möglich als Startwert eine Blutflussrate vorzugeben, mit der eine vorausgehende Behandlung begonnen halt. Die Vorgabe des Blutflusses zum Ende der Behandlung bietet aber dem Patienten eine erhöhte Sicherheit, da sich die Blutflussrate über den Verlauf der Behandlung auf Grund der Verdickung des Blutes im Allgemeinen verringert.

Die erfindungsgemäße Vorrichtung ermöglicht eine Maximierung des behandelten Blutvolumens innerhalb einer vorgegebenen Behandlungszeit, ohne dass es zu einer Schädigung des Blutes oder der Gefäße kommen kann. Dabei kann der Blutfluss dynamisch an die sich verändernden Bedingungen angepaßt werden. Insgesamt wird die Reinigungsleistung für kleine und große Moleküle optimiert. Mit der erfindungsgemäßen Vorrichtung

lässt sich auch die Anzahl von Alarme minimieren, insbesondere von Druckalarmen während der Blutbehandlung.

Die Auswahl der optimalen Blutflussrate wird dem Bedienungspersonal vorzugsweise signalisiert. Beispielsweise kann dem Personal auf einer Anzeigeeinheit angezeigt werden, auf welche der ermittelten charakteristischen Kenngrößen die Auswahl der Blutflussrate beruht. Dies ermöglicht eine Beobachtung von Veränderungen des Gefäßzugangs und eine Bewertung der Qualität der Punktion, so dass sich langfristige Trends im Hinblick auf das behandelte Blutvolumen aufzeigen lassen.

Im Folgenden wird ein Ausführungsbeispiel der Erfindung unter Bezugnahme auf die Zeichnungen näher erläutert.

Es zeigen:
- Fig. 1: die wesentlichen Komponenten der erfindungsgemäßen Blutbehandlungsvorrichtung mit der Einrichtung zum Vorgeben der Blutflussrate in stark vereinfachter schematischer Darstellung und
- Fig. 2: die zentrale Rechen- und Steuereinheit der erfindungsgemäßen Blutbehandlungsvorrichtung in vereinfachter schematischer Darstellung.

Die Blutbehandlungsvorrichtung, beispielsweise Hämodialysevorrichtung, weist einen Dialysator 1 auf, der durch eine semipermeable Membran 2 in eine Blutkammer 3 und eine Dialysierflüssigkeitskammer 4 getrennt ist. Der Einlass der Blutkammer 3 ist mit dem einen Ende einer Blutzuführleitung 5 verbunden, während der Auslass der Blutkammer 3 mit dem einen Ende einer Blutabführleitung 6 verbunden ist. Die anderen Enden der Blutzuführ- und -abführleitung 5, 6 sind mit einer arteriellen bzw. venösen Nadel 5', 6' verbunden, die an dem nicht dargestellten Gefäßzugang des Patienten angeschlossen werden. Die Blutzuführ- und -abführleitung 5, 6 stellen zusammen mit der Blutkammer 3 des Dialysators 1 den extrakorporalen Blutkreis I der Blutbehandlungsvorrichtung dar.

Das Dialysierflüssigkeitssystem II der Blütbehandlungsvorrichtung umfasst eine Einrichtung 7 zur Aufbereitung der Dialysierflüssigkeit, von der eine Dialysierflüssigkeitszuführleitung 8 abgeht, die zu der Dialysierflüssigkeitskammer 4 führt. Von der Dialysierflüssigkeitskammer 4 geht eine Dialysierflüssigkeitsabführleitung 9 ab, die zu einem Auslass 10 führt.

In der Blutzuführleitung 5 ist eine Blutpumpe 11 angeordnet, während in der Dialysierflüssigkeitsabführleitung 9 eine Dialysierflüssigkeitspumpe 12 angeordnet ist. Während der Blutbehandlung fördert die Blutpumpe 11 Blut von dem Patienten durch die arterielle Blutleitung 5 in die Blutkammer 3 und aus der Blutkammer 3 durch die venöse Blutleitung 6 zum Patienten.

Die Blutbehandlungsvorrichtung umfasst eine zentrale Steuer- und Recheneinheit 13, die über Steuerleitungen 11' und 12' mit der Blutpumpe 11 bzw. der Dialysierflüssigkeitspumpe 12 verbunden ist, um die Blutfluss- bzw. Dialysierflüssigkeitsrate einstellen zu können.

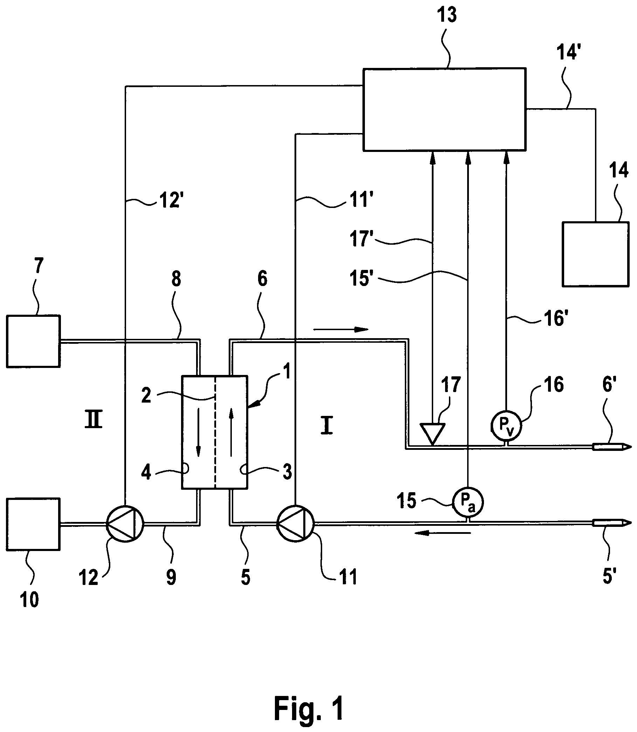

Während der Blutbehandlung werden verschiedene für die Blutbehandlung charakteristische Kenngrößen überwacht. Hierzu verfügt die Blutbehandlungsvorrichtung über mehrere Einrichtungen, mit der jeweils eine charakteristische Kenngröße (Parameter) ermittelt wird. Diese Einrichtungen sind in Fig. 1 nur schematisch dargestellt. Die dargestellten Einrichtungen sollen nur exemplarisch sein. Es ist grundsätzlich aber auch möglich, nur eine charakteristische Kenngröße zu überwachen.

Die Blutbehandlungsvorrichtung verfügt über eine Einrichtung 14 zur Ermittlung der Clearance K oder der Veränderung der Clearance ΔK bei einer Erhöhung des Blutflusses Q_{B}(t). Ein Verfahren und eine Vorrichtung zur Bestimmung der Clearance ist beispielsweise in der EP 0 845 273 A1 beschrieben. Die Clearance K erhöht sich mit der Erhöhung des Blutflusses. Die Erhöhung ist aber in der Regel nicht linear, da es mit einem erhöhten extrakorporalen Blutfluss auch zu einer erhöhten Rezirkulation des Blutes durch die Fistel des Patienten kommt. Die Clearance nähert sich hierdurch bei einem erhöhten Blutfluss einem Grenzwert an.

Neben der Einrichtung zur Bestimmung der Clearance weist die Blutbehandlungsvorrichtung eine Einrichtung 15 zum Ermitteln des arteriellen Drucks Pₐ(t) in der arteriellen Blutleitung 5 und eine Einrichtung 16 zum Ermitteln des venösen Drucks Pᵥ(t) in der venösen Blutleitung 6 auf. Arterieller und venöser Druck können maschinenseitig von entsprechenden Sensoren überwacht werden. Eine Einrichtung zum Messen des arteriellen und venösen Drucks ist beispielsweise aus der EP 2 383 004 A1 bekannt.

Durch das Ansaugen des Bluts mit der Blutpumpe 11 in die arterielle Blutleitung 5 wird das Blut einem Unterdruck gegenüber der Atmosphäre ausgesetzt, was zur Entgasung des Bluts führen kann. Hierbei entstehen im Blut gelöste Mikroblasen, die nicht vollständig aus dem Blut entfernt werden können und dem Patienten daher venös wieder zugeführt werden. Der Patient kann zwar ein bestimmtes Volumen an gelösten Mikroblasen gefahrlos verarbeiten. Bei Überschreiten eines Grenzwertes des infundierten akkumulierten Mikroblasenvolumens MES(t) besteht aber die Gefahr der Gefährdung des Patienten durch eine Embolie. Daher weist die Blutbehandlungsvorrichtung vorzugsweise auch eine Einrichtung 17 zur Ermittlung einer mit der Anzahl oder dem Volumen der im Blut vorhandenen Mikroblasen korrelierenden Kenngröße auf. Eine derartige Einrichtung ist beispielsweise in der WO 2006/128520 A1 beschrieben. Mit dieser Einrichtung kann die Häufigkeit des Auftretens von Embolie-Ereignissen analysiert werden.

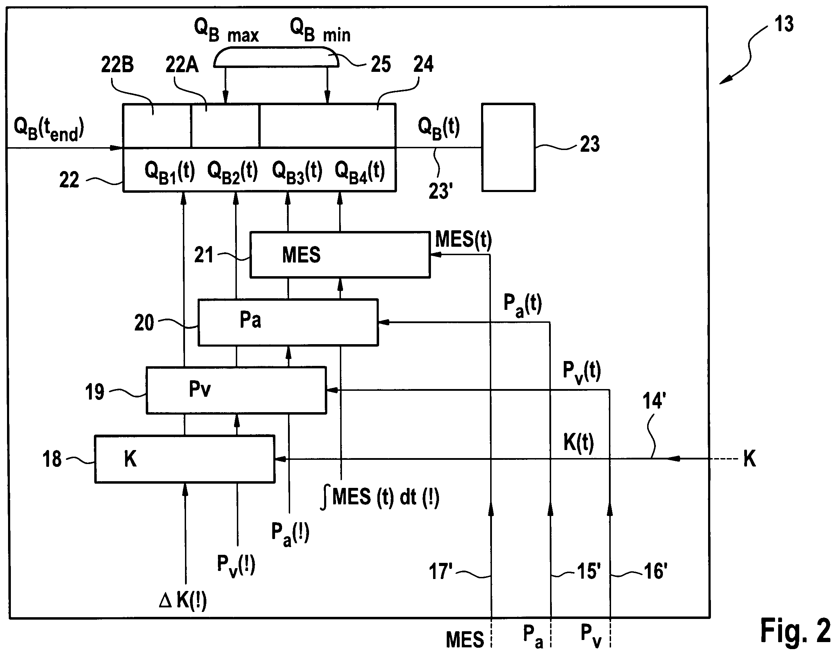

Nachfolgend wird unter Bezugnahme auf Fig. 2 die zentrale Rechen- und Steuereinheit 13 der Blutbehandlungsvorrichtung näher beschrieben.

Die ermittelten charakteristischen Kenngrößen empfängt die Rechen- und Steuereinheit 13 über Signalleitungen 14', 15', 16', 17', die mit den entsprechenden Einrichtungen 14, 15, 16, 17 zur Ermittlung der Kenngrößen verbunden sind.

Die Rechen- und Steuereinheit 13 weist für jede der charakteristischen Kenngrößen eine Einrichtung auf, die in Abhängigkeit von der ermittelten charakteristischen Kenngröße eine Blutflussrate Q_{Bn}(t) ermittelt. Diese Einrichtungen können Regeleinrichtungen sein, wobei die charakteristische Kenngröße die Regelgröße ist.

Die einzelnen Einrichtungen sind in Fig. 2 dargestellt. Die erste Regeleinrichtung 18 ermittelt eine erste Blutflussrate Q_{B1}(t) in Abhängigkeit von der Clearance(t) (K(t)), die zweite Regeleinrichtung 19 ermittelt eine zweite Blutflussrate Q_{B2}(t) in Abhängigkeit von dem venösen Druck Pᵥ(t), die dritte Regeleinrichtung 20 ermittelt einen dritten Blutfluss Q_{B3}(t) in Abhängigkeit von dem arteriellen Druck Pₐ(t) und die vierte Regeleinrichtung 21 ermittelt eine vierte Blutflussrate Q_{B4}(t) in Abhängigkeit von der für die Anzahl der Mikroblasen charakteristischen Kenngröße MES(t). Dabei vergleichen die Regeleinrichtungen 18 bis 21 die gemessenen Ist-Größen mit den vorgegebenen Sollwerten K(!), Pᵥ(!), Pₐ(!), ∫MES(t)dt(!). Derartige Regeleinrichtungen gehören zum Stand der Technik.

Die Anzahl der in Figur 2 dargestellten Regeleinrichtungen (18 bis 21) ist nur beispielhaft. Eine Ausführungsform kann nur eine Regeleinrichtung, entsprechend einer charakteristischen Kenngröße, umfassen. Eine andere Ausführungsform kann beliebig viele Regeleinrichtungen, entsprechend beliebig vielen charakteristischen Kenngrößen, umfassen.

Die Rechen- und Auswerteinheit 13 verfügt weiterhin über eine Einrichtung 22 zum Vorgeben einer Blutflussrate Q_{B}(t) aus den von den einzelnen Regeleinrichtungen 18 bis 21 vorgeschlagenen Blutflussraten Q_{Bn}(t). Die Einrichtung 22 zum Vorgeben einer Blutflussrate Q_{B}(t) wird nachfolgend auch als Auswahleinrichtung bezeichnet. Die Auswahl wird nach einem bestimmten Algorithmus getroffen.

Die Auswahleinrichtung 22 weist eine Datenverarbeitungseinheit 22A auf, auf der ein Datenverarbeitungsprogramm läuft. Die Datenverarbeitungseinheit 22A kann eine eigene Datenverarbeitungseinheit oder Teil einer Datenverarbeitungseinheit (Mikroprozessor) der zentralen Rechen- und Steuereinheit 13 sein. Die Datenverarbeitungseinheit ist derart programmiert, dass die Auswahl der Blutflussrate nach dem Algorithmus erfolgt.

Bei der Auswahl der Blutflussrate werden neben den dynamischen Kenngrößen Q_{B1}(t) bis Q_{B4}(t) noch zwei statische Kenngrößen berücksichtigt, die von einer Einrichtung 25 vorgegeben werden. Die Einrichtung 25 zur Vorgabe der statischen Kenngrößen kann eine Eingabeeinheit sein. Bei diesen Kenngrößen handelt es sich um einen Maximalwert Q_{B max} und einen Minimalwert Q_{B min} min für den Blutfluss Q_{B}(t).

Bei einer bevorzugten Ausführungsform vergleicht die Auswahleinrichtung 22 die vorgeschlagenen dynamischen Blutflüsse Q_{Bn}(t) und ermittelt den Blutfluss Q_{B}(t), der dem niedrigsten Wert der Blutflüsse entspricht. Dieser Wert wird dann mit dem Maximalwert Q_{B max} und dem Minimalwert Q_{B min} verglichen. Wenn der Wert Q_{B}(t) größer als der Minimalwert Q_{B min} und kleiner als der Maximalwert Q_{B max} ist, gibt die Einrichtung 22 als Blutflussrate den Wert Q_{B}(t) vor. Dieser Wert verändert sich über den Verlauf der Blutbehandlung in Abhängigkeit von den gemessenen charakteristischen Kenngrößen.

Auf der Grundlage des vorgegebenen Algorithmus erfolgt also während der Blutbehandlung die Regelung des Blutflusses in Abhängigkeit von einer oder mehreren der charakteristischen Kenngrößen. Es ist also möglich, dass in einem Zeitintervall der Blutbehandlung die Regelung mit beispielsweise dem arteriellen und/oder dem venösen Druck Pₐ(t), Pᵥ(t) als Regelgröße und in einem anderen Zeitintervall beispielsweise mit der Clearance(t) als Regelgröße erfolgt. Folglich können sich die Regelgrößen laufend ändern.

Bei dem vorliegenden Ausführungsbeispiel analysiert die Regeleinrichtung 21 die Häufigkeit des Auftretens von Embolie-Ereignissen und regelt den Blutfluss Q_{B}(t) so, dass die Häufigkeit der Embolie-Ereignisse unterhalb eines bestimmten Grenzwertes liegt. Die Regeleinrichtung 20 regelt den Blutfluss Q_{B}(t), so dass der eingestellte Soll-Wert Pₐ(!) für den arteriellen Druck Pₐ(t) erreicht wird, während die Regeleinrichtung 19 den venösen Druck Pᵥ(t) regelt, so dass der Ist-Wert Pᵥ(t) dem Soll-Wert Pᵥ(!) entspricht. Die Regeleinrichtung 18 versucht, den Einfluss der mit einer Erhöhung des Blutflusses zunehmenden Rezirkulation zwischen arteriellem und venösem Zugang zu begrenzen.

Darüber hinaus ist eine Anzeigeeinheit 23 vorgesehen, die über eine Signalleitung 23' mit der Einrichtung 22 zur Auswahl der Blutflussrate Q_{B}(t) verbunden ist. Auf der Anzeigeeinheit 23 wird dem Bedienpersonal signalisiert, welche der charakteristischen Kenngrößen für die Regelung momentan bestimmend ist. Das Bedienpersonal kann also während der Blutbehandlung überwachen, ob die Regelung beispielsweise auf der Grundlage des arteriellen und/oder venösen Drucks P_{a,v}(t) oder der Clearance oder der Embolie-Ereignisse MES erfolgt. Die Signalisierung auf der Anzeigeeinheit 23 kann mit den bekannten optischen und/oder akustischen Mitteln erfolgen.

Zu Beginn der Blutbehandlung kann vom Bedienpersonal ein fester Wert für den Blutfluss Q_{B}(t) vorgegeben werden, der sich dann im Laufe der Blutbehandlung automatisch ändert. Eine bevorzugte Ausführungsform sieht aber vor, dass die Datenverarbeitungseinheit 22 einen Speicher 24 aufweist, in dem als Startwert für eine nachfolgende Blutbehandlung eines Patienten der patientenspezifische Wert des Blutflusses am Ende einer vorausgehenden Blutbehandlung des Patienten gespeichert ist. Der Wert wird zu Beginn der Blutbehandlung aus dem Speicher 24 ausgelesen und als Startwert für die neue Behandlung vorgegeben. In dem Speicher 24 ist eine Vielzahl von Werten für eine Vielzahl von Patienten abgelegt, die zu Beginn der einzelnen Blutbehandlungen aufgerufen werden können.

## Patentansprüche

1. Vorrichtung zur extrakorporalen Blutbehandlung, bei der Blut in einem extrakorporalen Blutkreislauf (I) durch eine arterielle Blutleitung (5) in die Blutkammer (2) eines durch eine semipermeable Membran (2) in die Blutkammer (2) und eine Dialysierflüssigkeitskammer (3) getrennten Dialysators (1) strömt und durch eine venöse Blutleitung (6) aus der Blutkammer (2) strömt, mit
Mitteln (11) zum Fördern von Blut im extrakorporalen Blutkreislauf mit einer vorgegebenen Blutflussrate,
einer Einrichtung (22) zum Vorgeben der Blutflussrate, mit der die Mittel (11) zum Fördern von Blut im extrakorporalen Blutkreislauf (I) betrieben werden, so dass Blut mit der vorgegebenen Blutflussrate durch den extrakorporalen Blutkreislauf strömt,
mindestens zwei Einrichtungen (14, 15, 16, 17) zum Ermitteln von mindestens zwei für die extrakorporale Blutbehandlung charakteristischen Kenngrößen,
mindestens zwei Einrichtungen (18, 19, 20, 21) zum Ermitteln von mindestens zwei Blutflussraten in Abhängigkeit von den mindestens zwei für die extrakorporale Blutbehandlung charakteristischen Kenngrößen,
**dadurch gekennzeichnet, dass**
die Einrichtung (22) zum Vorgeben der Blutflussrate eine Datenverarbeitungseinheit (22A) aufweist, die derart programmiert ist, dass auf der Grundlage von einem vorgegebenen Algorithmus aus den mindestens zwei Blutflussraten Q_{Bn}(t), die von den mindestens zwei Einrichtungen (18,19, 20, 21) zum Ermitteln von mindestens zwei Blutflussraten in Abhängigkeit von den mindestens zwei für die extrakorporale Blutbehandlung charakteristischen Kenngrößen ermittelt werden, eine Blutflussrate Q_{β}(t) ausgewählt wird, mit der die Mittel (11) zum Fördern von Blut irm extrakorporalen Blutkreislauf betrieben werden.

2. Vorrichtung zur extrakorporalen Blutbehandlung nach Anspruch 1, **dadurch gekennzeichnet, dass** die Datenverarbeitungseinheit (22A) der Einrichtung (22) zum Vorgeben der Blutflussrate derart programmiert ist, dass der vorgegebene Algorithmus zur Auswahl der Blutflussrate ein Auswahlkriterium vorsieht, dass darin liegt, den niedrigsten Wert der Blutflussraten auszuwählen.

3. Vorrichtung zur extrakorporalen Blutbehandlung nach Anspruch 1, **dadurch gekennzeichnet, dass** die Blutbehandlungsvorrichtung eine Einrichtung (25) zum Vorgeben eines Maximalwertes und/oder eines Minimalwertes für die Blutflussrate aufweist, wobei
die Datenverarbeitungseinheit (22A) der Einrichtung (22) zum Vorgeben der Blutflussrate derart programmiert ist, dass
in Abhängigkeit von dem vorgegebenen Algorithmus aus den mindestens zwei Blutflussraten, die von den mindestens zwei Einrichtungen (18, 19, 20, 21) zum Ermitteln von mindestens zwei Blutflussraten in Abhängigkeit von den mindestens zwei für die extrakorporale Blutbehandlung charakteristischen Kenngrößen ermittelt werden, und
in Abhängigkeit von der maximalen und/oder minimalen Blutflussrate, die von der Einrichtung (25) zum Vorgeben eines Maximalwertes oder Minimalwertes für die Blutflussrate vorgegeben wird, eine Blutflussrate ausgewählt wird, mit der die Mittel (11) zum Fördern von Blut im extrakorporalen Blutkreislauf betrieben werden.

4. Vorrichtung zur extrakorporalen Blutbehandlung nach einem der Ansprüche 1 bis 3, **dadurch gekennzeichnet, dass** die Datenverarbeitungseinheit (22A) der Einrichtung (22) zum Vorgeben der Blutflussrate eine Speichereinheit (22B) aufweist, in der die zum Ende einer vorausgehenden Blutbehandlung vorgegebene Blutflussrate speicherbar ist, wobei die Datenverarbeitungseinheit (22A) derart programmiert ist, dass zu Beginn einer nachfolgenden Blutbehandlung als Startwert für die nachfolgende Blutbehandlung die in der Speichereinheit (22B) gespeicherte Blutflussrate der vorausgehenden Blutbehandlung vorgegeben wird.

5. Vorrichtung zur extrakorporalen Blutbehandlung nach einem der Ansprüche 2 bis 4, **dadurch gekennzeichnet,**
**dass** die mindestens zwei Einrichtungen (14, 15, 16, 17) zum Ermitteln von mindestens zwei für die extrakorporale Blutbehandlung charakteristischen Kenngrößen eine Einrichtung (15) zum Messen des arteriellen Drucks in der arteriellen Blutleitung aufweisen, wobei die mindestens zwei Einrichtungen (18,19,20, 21) zum Ermitteln von mindestens zwei Blutflussraten in Abhängigkeit von den mindestens zwei für die extrakorporale Blutbehandlung charakteristischen Kenngrößen eine Einrichtung (20) zum Ermitteln einer Blutflussrate in Abhängigkeit von dem arteriellen Druck aufweisen,
und/oder
**dass** die mindestens zwei Einrichtungen (14, 15, 16, 17) zum Ermitteln von mindestens zwei für die extrakorporale Blutbehandlung charakteristischen Kenngrößen eine Einrichtung (14) zum Messen des venösen Drucks in der venösen Blutleitung aufweisen, wobei die mindestens zwei Einrichtungen (18, 19, 20, 21) zum Ermitteln von mindestens zwei Blutflussraten in Abhängigkeit von den mindestens zwei für die extrakorporale Blutbehandlung charakteristischen Kenngrößen eine Einrichtung (18) zum Ermitteln einer Blutflussrate in Abhängigkeit von dem venösen Druck aufweisen.

6. Vorrichtung zur extrakorporalen Blutbehandlung nach einem der Ansprüche 1 bis 5, **dadurch gekennzeichnet, dass** die mindestens zwei Einrichtungen (14, 15, 16, 17) zum Ermitteln von mindestens zwei für die extrakorporale Blutbehandlung charakteristischen Kenngrößen eine Einrichtung (14) zum Bestimmen der Clearance aufweisen, wobei die mindestens zwei Einrichtungen (18, 19, 20, 21) zum Ermitteln von mindestens zwei Blutflussraten in Abhängigkeit von den mindestens zwei für die extrakorporale Blutbehandlung charakteristischen Kenngrößen eine Einrichtung (20) zum Ermitteln einer Blutflussrate in Abhängigkeit von der Clearance aufweisen.

7. Vorrichtung zur extrakorporalen Blutbehandlung nach einem der Ansprüche 1 bis 6, **dadurch gekennzeichnet, dass** die mindestens zwei Einrichtungen (14, 15, 16, 17) zum Ermitteln von mindestens zwei für die extrakorporale Blutbehandlung charakteristischen Kenngrößen eine Einrichtung (17) zum Bestimmen einer für die Anzahl der in dem Blut enthaltenden Mikroblasen charakteristischen Größe aufweisen, wobei die mindestens zwei Einrichtungen (18, 19, 20, 21) zum Ermitteln von mindestens zwei Blutflussraten in Abhängigkeit von den mindestens zwei für die extrakorporale Blutbehandlung charakteristischen Kenngrößen eine Einrichtung (21) zum Ermitteln einer Blutflussrate in Abhängigkeit von der für die Anzahl der in dem Blut enthaltenden Mikroblasen charakteristischen Größe aufweisen.

8. Vorrichtung zur extrakorporalen Blutbehandlung nach einem der Ansprüche 1 bis 7, **dadurch gekennzeichnet, dass** eine Anzeigeeinheit (23) vorgesehen ist, wobei die Datenverarbeitungseinheit (22A) der Einrichtung (22) zum Vorgeben der Blutflussrate derart programmiert ist, dass auf der Anzeigeeinheit (23) die charakteristische Kenngröße angezeigt wird, auf deren Grundlage die Blutflussrate vorgegeben wird.

## Claims

1. Apparatus for extra-corporeal blood treatment in which, in an extra-corporeal blood circuit (I), blood flows through an arterial blood line (5) into the blood chamber (2) of a dialyser (1) which is divided into the blood chamber (2) and a dialysis-fluid chamber (3) by a semi-permeable membrane (2), and flows out of the blood chamber (2) through a venous blood line (6), comprising
means (11) for pumping blood in the extra-corporeal blood circuit at a preset blood flow rate,
an arrangement (22) for presetting the blood flow rate at which the means (11) for pumping blood in the extra-corporeal blood circuit (I) are operated, thus causing blood to flow through the extra-corporeal blood circuit at the preset blood flow rate,
at least two arrangements (14, 15, 16, 17) for determining at least two parameter characteristics of the extra-corporeal blood treatment, and
at least two arrangements (18, 19, 20, 21) for determining at least two blood flow rates as a function of the at least two parameter characteristics of the extra-corporeal blood treatment,
**characterised in that**
the arrangement (22) for presetting the blood flow rate has a data processing unit (22A) which is programmed in such a way that there is selected, on the basis of a preset algorithm, from the at least two blood flow rates Q_{Bn}(t) which are determined by the at least two arrangements (14, 15, 16, 17) for determining at least two blood flow rates as a function of the at least two parameter characteristics of the extra-corporeal blood treatment, a blood flow rate Q_{B}(t) at which the means (11) for pumping blood in the extra-corporeal blood circuit are operated.

2. Apparatus for extra-corporeal blood treatment according to claim 1, **characterised in that** the data processing unit (22A) of the arrangement (22) for presetting the blood flow rate is programmed in such a way that the preset algorithm for selecting the blood flow rate provides a criteria for selection that will be to select the lowest value from the blood flow rates.

3. Apparatus for extra-corporeal blood treatment according to claim 1, **characterised in that** the blood treatment apparatus has an arrangement (25) for presetting a maximum value and/or a minimum value for the blood flow rate,
the data processing unit (22A) of the arrangement (22) for presetting the blood flow rate being programmed in such a way that
a blood flow rate at which the means (11) for pumping blood in the extra-corporeal blood circuit are operated is selected, as a function of the preset algorithm from the at least two blood flow rates which are determined by the at least two arrangements (18, 19, 20, 21) for determining at least two blood flow rates as a function of the at least two parameter characteristics of the extra-corporeal blood treatment and as a function of the maximum and/or minimum value which is preset by the arrangement (25) for presetting a maximum and/or minimum value for the blood flow rate.

4. Apparatus for extra-corporeal blood treatment according to one of claims 1 to 3, **characterised in that** the data processing unit (22A) of the arrangement (22) for presetting the blood flow rate has a memory unit (22B) in which the blood flow rate which was preset at the end of a preceding blood treatment can be stored, the data processing unit (22A) being programmed in such a way that the blood flow rate from the preceding blood treatment which is stored in the memory unit (22B) is preset as a starting value for a subsequent blood treatment at the beginning of the subsequent blood treatment.

5. Apparatus for extra-corporeal blood treatment according to one of claims 1 to 4, **characterised in that**
the at least two arrangements (14, 15, 16, 17) for determining at least two parameter characteristic of the extra-corporeal blood treatment have an arrangement (15) for measuring the arterial pressure in the arterial blood line, the at least two arrangements (18, 19, 20, 21) for determining at least two blood flow rate as a function of the at least two parameter characteristics of the extra-corporeal blood treatment having an arrangement (20) for determining a blood flow rate as a function of the arterial pressure, and/or
the at least two arrangements (14, 15, 16, 17) for determining at least two parameter characteristics of the extra-corporeal blood treatment have an arrangement (14) for measuring the venous pressure in the venous blood line, the at least two arrangements (18, 19, 20, 21) for determining at least two blood flow rates as a function of the at least two parameter characteristic of the extra-corporeal blood treatment having an arrangement (18) for determining a blood flow rate as a function of the venous pressure.

6. Apparatus for extra-corporeal blood treatment according to one of claims 1 to 5, **characterised in that** the at least two arrangements (14, 15, 16, 17) for determining at least two parameter characteristics of the extra-corporeal blood treatment have an arrangement (14) for determining clearance, the at least two arrangements (18, 19, 20, 21) for determining at least two blood flow rates as a function of the at least two parameter characteristics of the extra-corporeal blood treatment having an arrangement (20) for determining a blood flow rate as a function of clearance.

7. Apparatus for extra-corporeal blood treatment according to one of claims 1 to 6, **characterised in that** the at least two arrangements (14, 15, 16, 17) for determining at least two parameter characteristics of the extra-corporeal blood treatment have an arrangement (17) for determining a parameter characteristic of the number of micro-bubbles contained in the blood, the at least two arrangements (18, 19, 20, 21) for determining at least two blood flow rates as a function of at least two parameter characteristics of the extra-corporeal blood treatment having an arrangement (21) for determining a blood flow rate as a function of the parameter characteristic of the number of micro-bubbles contained in the blood.

8. Apparatus for extra-corporeal blood treatment according to one of claims 1 to 7, **characterised in that** an indicator unit (23) is provided, the data processing unit (22A) of the arrangement (22) for presetting the blood flow rate being programmed in such a way that the characteristic parameter on the basis of which the blood flow rate is preset is indicated on the indicator unit (23).

## Revendications

1. Dispositif servant au traitement de sang extracorporel, dans lequel le sang s'écoule dans un circuit de sang (I) extracorporel, à travers une conduite de sang (5) artérielle, dans la chambre de sang (2) d'un dialyseur (1) séparé par une membrane (2) semi-perméable en la chambre de sang (2) et en une chambre de liquide de dialyse (3) et s'écoule par une conduite de sang (6) veineuse hors de la chambre de sang (2), avec
des moyens (11) servant à refouler du sang dans le circuit de sang extracorporel à un débit sanguin spécifié,
un système (22) servant à spécifier le débit sanguin, lequel permet de faire fonctionner les moyens (11) servant à refouler du sang dans le circuit de sang (I) extracorporel de sorte que le sang s'écoule selon le débit sanguin spécifié à travers le circuit de sang extracorporel,
au moins deux systèmes (14, 15, 16, 17) servant à déterminer au moins deux grandeurs caractéristiques pour le traitement de sang extracorporel,
au moins deux systèmes (18, 19, 20, 21) servant à déterminer au moins deux débits sanguins en fonction des au moins deux grandeurs caractéristiques pour le traitement de sang extracorporel,
**caractérisé en ce que**
le système (22) servant à spécifier le débit sanguin présente une unité de traitement de données (22A), qui est programmée de telle manière que sur la base d'un algorithme spécifié issu des au moins deux débits sanguins Q_{Bn}(t), qui sont déterminés par les au moins deux systèmes (18, 19, 20, 21) servant à déterminer au moins deux débits sanguins en fonction des au moins deux grandeurs caractéristiques pour le traitement de sang extracorporel, un débit sanguin Q_{B}(t) est sélectionné, qui permet de faire fonctionner les moyens (11) servant à refouler du sang dans le circuit de sang extracorporel.

2. Dispositif servant au traitement de sang extracorporel selon la revendication 1, **caractérisé en ce que** l'unité de traitement de données (22A) du système (22) servant à spécifier le débit sanguin est programmée de telle manière que l'algorithme spécifié servant à sélectionner le débit sanguin prévoit un critère de sélection, qui consiste à sélectionner la valeur la plus basse des débits sanguins.

3. Dispositif servant au traitement de sang extracorporel selon la revendication 1, **caractérisé en ce que** le dispositif de traitement de sang présente un système (25) servant à spécifier une valeur maximale et/ou une valeur minimale pour le débit sanguin, dans lequel l'unité de traitement de données (22A) du système (22) servant à spécifier le débit sanguin est programmée de telle manière
qu'en fonction de l'algorithme spécifié issu des au moins deux débits sanguins spécifiés, qui sont déterminés par les au moins deux systèmes (18, 19, 20, 21) servant à déterminer au moins deux débits sanguins en fonction des au moins deux grandeurs caractéristiques pour le traitement de sang extracorporel, et
qu'en fonction du débit sanguin maximal et/ou minimal, qui est spécifié par le système (25) servant à spécifier une valeur maximale ou une valeur minimale pour le débit sanguin, un débit sanguin est sélectionné, lequel permet de faire fonctionner les moyens (11) servant à refouler du sang dans le circuit de sang extracorporel.

4. Dispositif servant au traitement de sang extracorporel selon l'une quelconque des revendications 1 à 3, **caractérisé en ce que** l'unité de traitement de données (22A) du système (22) servant à spécifier le débit sanguin présente une unité de mémoire (22B), dans laquelle le débit sanguin spécifié jusqu'à la fin d'un traitement de sang précédent peut être mémorisé, dans lequel l'unité de traitement de données (22A) est programmée de telle manière que le débit sanguin mémorisé dans l'unité de mémoire (22B) du traitement de sang précédent est spécifié au début d'un traitement de sang subséquent comme valeur de départ pour le traitement de sang subséquent.

5. Dispositif servant au traitement de sang extracorporel selon l'une quelconque des revendications 2 à 4, **caractérisé en ce**
**que** les au moins deux systèmes (14, 15, 16, 17) servant à déterminer au moins deux grandeurs caractéristiques pour le traitement de sang extracorporel présentent un système (15) servant à mesurer la pression artérielle dans la conduite de sang artérielle, dans lequel les au moins deux systèmes (18, 19, 20, 21) servant à déterminer au moins deux débits sanguins en fonction des au moins deux grandeurs caractéristiques pour le traitement de sang extracorporel présentent un système (20) servant à déterminer un débit sanguin en fonction de la pression artérielle,
et/ou
**que** les au moins deux systèmes (14, 15, 16, 17) servant à déterminer au moins deux grandeurs caractéristiques pour le traitement de sang extracorporel présentent un système (14) servant à mesurer la pression veineuse dans la conduite de sang veineuse, dans lequel les au moins deux systèmes (18, 19, 20, 21) servant à déterminer au moins deux débits sanguins en fonction des au moins deux grandeurs caractéristiques pour le traitement de sang extracorporel présentent un système (18) servant à déterminer un débit sanguin en fonction de la pression veineuse.

6. Dispositif servant au traitement de sang extracorporel selon l'une quelconque des revendications 1 à 5, **caractérisé en ce que** les au moins deux systèmes (14, 15, 16, 17) servant à déterminer au moins deux grandeurs caractéristiques pour le traitement de sang extracorporel présentent un système (14) servant à déterminer la clairance, dans lequel les au moins deux systèmes (18, 19, 20, 21) servant à déterminer au moins deux débits sanguins en fonction des au moins deux grandeurs caractéristiques pour le traitement de sang extracorporel présentent un système (20) servant à déterminer un débit sanguin en fonction de la clairance.

7. Dispositif servant au traitement de sang extracorporel selon l'une quelconque des revendications 1 à 6, **caractérisé en ce que** les au moins deux systèmes (14, 15, 16, 17) servant à déterminer au moins deux grandeurs caractéristiques pour le traitement de sang extracorporel présentent un système (17) servant à définir une grandeur caractéristique du nombre des microbulles contenues dans le sang, dans lequel les au moins deux systèmes (18, 19, 20, 21) servant à déterminer au moins deux débits sanguins en fonction des au moins deux grandeurs caractéristiques pour le traitement de sang extracorporel présentent un système (21) servant à déterminer un débit sanguin en fonction de la grandeur caractéristique du nombre des microbulles contenues dans le sang.

8. Dispositif servant au traitement de sang extracorporel selon l'une quelconque des revendications 1 à 7, **caractérisé en ce qu'**une unité d'affichage (23) est prévue, dans lequel l'unité de traitement de données (22A) du système (22) servant à spécifier le débit est programmée de telle manière que la grandeur caractéristique, sur la base de laquelle le débit sanguin est spécifié, est affichée sur l'unité d'affichage (23).
